# EUROPEAN PATENT SPECIFICATION

(11) **EP 3 020 405 B1**
(45) Date of publication and mention of the grant of the patent: **08.09.2021**
(21) Application number: 14823610.2
(22) Date of filing: 04.07.2014
(51) Int. Cl.: A61K 35/28, A61K 35/545, A61P 1/16

(54) **COMPOSITION FOR PREVENTING AND TREATING LIVER FIBROSIS OR LIVER CIRRHOSIS,CONTAINING, AS ACTIVE INGREDIENT, MESENCHYMAL STEM CELLS DERIVED FROM HUMAN EMBRYONIC STEM CELLS**
ZUSAMMENSETZUNG ZUR VORBEUGUNG UND BEHANDLUNG VON LEBERFIBROSE ODER LEBERZIRRHOSE MIT MESENCHYMALEN STAMMZELLEN AUS MENSCHLICHEN EMBRYONALEN STAMMZELLEN ALS WIRKSTOFF
COMPOSITION DESTINÉE À LA PRÉVENTION ET AU TRAITEMENT DE LA FIBROSE DU FOIE OU DE LA CIRRHOSE DU FOIE, LADITE COMPOSITION CONTENANT, COMME PRINCIPE ACTIF, DES CELLULES SOUCHES MÉSENCHYMATEUSES DÉRIVÉES DE CELLULES SOUCHES EMBRYONNAIRES HUMAINES

(30) Priority: 11.07.2013 KR 20130081448; 03.07.2014 KR 20140082822
(43) Date of publication of application: 18.05.2016
(73) Proprietor: Seoul National University Hospital, Seoul 110-744 (KR)
(72) Inventor: LEE, Eun Ju, Seoul 136-771 (KR); KIM, Hyo Soo, Seoul 140-210 (KR)
(74) Representative: dompatent von Kreisler Selting Werner - Partnerschaft von Patent- und Rechtsanwälten mbB
(86) International application number: PCT/KR2014/005979
(87) International publication number: WO 2015/005614

(56) References cited:
- EP-A1- 2 495 311
- WO-A1-2013/055297
- WO-A1-2013/082543
- KR-A- 20100 074 386
- US-A1- 2012 276 625
- PEIMAN HEMATTI: "Human embryonic stem cell-derived mesenchymal stromal cells", TRANSFUSION., vol. 51, 1 November 2011 (2011-11-01), pages 138S-144S, XP055327626, US ISSN: 0041-1132, DOI: 10.1111/j.1537-2995.2011.03376.x
- TRIVEDI ET AL: "Derivation and immunological characterization of mesenchymal stromal cells from human embryonic stem cells", EXPERIMENTAL HEMATOLOGY, ELSEVIER INC, US, vol. 36, no. 3, 7 January 2008 (2008-01-07), pages 350-359, XP022480056, ISSN: 0301-472X
- SHUFANG ZHANG ET AL: "Neonatal Desensitization Supports Long-Term Survival and Functional Integration of Human Embryonic Stem Cell-Derived Mesenchymal Stem Cells in Rat Joint Cartilage Without Immunosuppression", STEM CELLS AND DEVELOPMENT, vol. 22, no. 1, 12 July 2012 (2012-07-12), pages 90-101, XP055327753, NL ISSN: 1547-3287, DOI: 10.1089/scd.2012.0116
- WENCHUAN CHEN ET AL: "Human Embryonic Stem Cell-Derived Mesenchymal Stem Cell Seeding on Calcium Phosphate Cement-Chitosan-RGD Scaffold for Bone Repair", TISSUE ENGINEERING PART A, vol. 19, no. 7-8, 1 April 2013 (2013-04-01), pages 915-927, XP055327755, US ISSN: 1937-3341, DOI: 10.1089/ten.tea.2012.0172
- TOMONARI AWAYA ET AL: "Selective Development of Myogenic Mesenchymal Cells from Human Embryonic and Induced Pluripotent Stem Cells", PLOS ONE, vol. 7, no. 12, 7 December 2012 (2012-12-07), page e51638, XP055327758, DOI: 10.1371/journal.pone.0051638
- TAN Z ET AL: "Immunomodulative effects of mesenchymal stem cells derived from human embryonic stem cells in vivo and in vitro", ZHEJIANG UNIVERSITY. JOURNAL. SCIENCE B: INTERNATIONAL BIOMEDICINE & BIOTECHNOLOGY JOURNAL, ZHEIJIANG UNIVERSITY PRESS, CN, vol. 12, no. 1, 1 January 2011 (2011-01-01), pages 18-27, XP002744077, ISSN: 1673-1581, DOI: 10.1631/JZUS.B1000074
- JUHA P. LAURILA ET AL: "Human embryonic stem cell-derived mesenchymal stromal cell transplantation in a rat hind limb injury model", CYTOTHERAPY, vol. 11, no. 6, 1 January 2009 (2009-01-01), pages 726-737, XP055327767, GB ISSN: 1465-3249, DOI: 10.3109/14653240903067299
- HEMATTI PEIMAN: "Human embryonic stem cell-derived mesenchymal progenitors: an overview", METHODS IN MOLECULAR BIOLOGY,, vol. 690, 1 January 2011 (2011-01-01), pages 163-174, XP009161182, ISSN: 1940-6029 ISBN: 978-1-61779-291-5
- ZHANG, Z. ET AL.: 'Human umbilical cord mesenchymal stem cells improve liver function and ascites in decompensated liver cirrhosis patients' JOURNAL OF GASTROENTEROLOGY AND HEPATOLOGY vol. 27, no. 2, 2012, pages 112 - 120, XP055312654
- ZHAO, D.-C. ET AL.: 'Bone marrow-derived mesenchymal stem cells protect against experimental liver fibrosis in rats' WORLD JOURNAL OF GASTROENTEROLOGY vol. 11, no. 22, 2005, pages 3431 - 3440, XP002566970
- ABDEL AZIZ, M. T. ET AL.: 'Therapeutic potential of bone marrow-derived mesenchymal stem cells on experimental liver fibrosis' CLINICAL BIOCHEMISTRY vol. 40, 2007, pages 893 - 899, XP022162317
- LE BLANC, K. ET AL.: 'Mesenchymal stem cells for treatment of steroid -resistant, severe, acute graft-versus-host disease: a phase II study' THE LANCET vol. 371, 2008, pages 1579 - 1586, XP026859188
- Seok Jin Kim ET AL: "Hematopoietic Differentiation of Embryoid Bodies Derived from the Human Embryonic Stem Cell Line SNUhES3 in Co-culture with Human Bone Marrow Stromal Cells", Yonsei Medical Journal, vol. 46, no. 5, 1 January 2005 (2005-01-01), pages 693-699, XP055489314,
- Eun Ju Lee ET AL: "Novel Embryoid Body-Based Method to Derive Mesenchymal Stem Cells from Human Embryonic Stem Cells", Tissue Engineering Part A, vol. 16, no. 2, 1 February 2010 (2010-02-01), pages 705-715, XP055100944, ISSN: 1937-3341, DOI: 10.1089/ten.tea.2008.0596
- Anonymous: "Clonetics(TM) Immortalized Human Dermal Blood & Lymphatic Microvascular Endothelial Cell Systems", , 1 January 2013 (2013-01-01), pages 1-6, XP055635007, Retrieved from the Internet: URL:https://bioscience.lonza.com/lonza_bs/ CH/en/download/product/asset/27640 [retrieved on 2019-10-23]

## Description

### TECHNICAL FIELD

The present invention relates to a composition for use in preventing and treating liver fibrosis and liver cirrhosis, which contains mesenchymal stem cells derived from human embryonic stem cells as an active ingredient.

### BACKGROUND ART

The liver is an organ that performs various functions. In recent years, the development of liver diseases and the number of deaths caused by liver diseases have gradually increased. Among liver disease patients in Korea, hepatitis B or hepatitis C patients were many in the past, but alcoholic liver disease patients rather than infectious liver disease patients are currently increasing, like liver disease patients in the Europe and America. In addition, as women who are socially active are increasing, women's opportunity for drinking is also increasing, and thus women with alcoholic liver disease are also increasing compared to previous years. Due to this tendency, studies on agents for treating liver diseases have been actively conducted worldwide.

Generally, liver cirrhosis refers to the end stage of liver disease. The major causes of liver cirrhosis are diverse, including hepatitis, viral infections, alcohol intoxication, bile acid secretion disorder, drug addiction, allergy, and excessive iron deposition.

Liver cells can be repaired by regeneration owing to their strong regenerating ability even when they are destroyed to some degree. However, after a certain point of time after liver cell destruction, the destroyed cells are not regenerated but undergo fibrosis, which results in liver hardening. This condition, in which the liver is hardened by a change in its structure so that it cannot go back to the original state, is referred to as liver cirrhosis.

The process of liver fibrosis leading to liver cirrhosis is a reaction occurring in response to continuous stimulation, and can be divided into the following three steps, similar to the wound healing process: 1) acute inflammation, 2) synthesis of extracellular matrix (ECM) components including collagen, and 3) tissue reconstruction (scar formation) (Ramadori R, Knittel R, and Saile B. (1998) Fibrosis and Altered Matrix Synthesis Digestion 59:372-375). The extracellular matrix is composed of collagen, matrix glycoproteins (fibronectin, laminin, etc.), proteoglycan and the like, but is composed mainly of collagen. After liver cells are destroyed, the liver tissue is reconstructed into new liver cells. At this time, if the cells fail to constitute perfect cellular structures such as cytoplasm and extracellular membrane and only the extracellular membrane which supports the cellular skeleton structure is developed, collagen that is the main component of the extracellular matrix of liver cells is excessively deposited, and the liver tissue is hardened by fibrotic collagen, resulting in liver cirrhosis.

Liver fibrosis refers to a disease in which liver tissue in a chronic inflammatory state is repeatedly damaged and repaired so that connective tissues such as collagen are excessively deposited in the liver tissue, thereby causing scars in the liver tissue.

Generally, unlike liver cirrhosis, liver fibrosis is reversible and is composed of thin fibrils without nodule formation. Once the cause of hepatic injury is eliminated, the liver can be returned to the normal state. However, if the liver fibrosis mechanism is continuously repeated, the liver fibrosis leads to irreversible liver cirrhosis in which crosslinking between connective tissues increases to accumulate thick fibrils, and a liver lobule loses its normal structure to cause nodule formation.

Liver diseases are caused by various causes, but if these liver diseases become chronic, they commonly lead to liver fibrosis or liver cirrhosis regardless of the causes thereof. Liver diseases are asymptomatic in the initial stage, and thus are difficult to diagnose early. Furthermore, because liver diseases are generally found in the chronic stage, these liver diseases are not easy to treat and have a high mortality rate, and thus pose social problems. In addition, therapeutic agents having excellent effects have not yet been developed.

As is known in the art, liver fibrosis mechanisms are induced by complex interactions between cells, cytokines and extracellular matrix (ECM). The excessive production of ECM in liver fibrosis is caused because activation of hepatic stellate cells (HSCs) is promoted by various cytokines that are secreted due to activation of macrophage Kupffer cells in the liver and because the production of connective tissues in the activated hepatic stellate cells is increased.

Hepatic stellate cells were observed by Kupffer in 1876, described by Ito, and established by Wake in 1971. Hepatic stellate cells are located in the space of Disse between the sinusoidal endothelial cells and hepatic epithelial cells were named in various ways such as hepatic stellate cells because of their stellate shape, Ito cells, vitamin A storing cells because of having lipid droplets containing vitamin A, fat storing cells, or perisinusoidal hepatic lipocytes, but were formally termed "hepatic stellate cells (HSCs)" in the late 1990s, and this term has been used to date.

Kupffer cells are activated either by phagocytosis of hepatic cells damaged by various toxic substances or directly by toxic substances to secrete cytokines such as transforming growth factor-β (TGF-beta), platelet-derived growth factor (PDGF) and the like, and are activated again by these cytokines in an autocrine manner. In addition, sinusoidal endothelial cells and hepatic stellate cells in liver tissue are activated by cytokines secreted by Kupffer cells. The normal ratio of extracellular matrix (ECM) components is broken by type IV collagenase secreted by activated hepatic stellate cells and increased collagen production while basement membrane ECM (basement membrane-like matrix, for example, type IV collagen) is degraded, and interstitial ECMs (interstitial type matrices, for example, type I collagen and type III collagen) are entangled with each other to form fibril-forming collagen, and then accumulated in the space of Disse. Hepatic stellate cells are activated by the degraded basement membrane ECM and the accumulated interstitial ECM.

In addition, activated hepatic stellate cells also inhibit the degradation of interstitial ECM by stimulating the production of macroglobulin and TIMP, which are inhibitors of metalloproteinase (MMP) activity. Because the mass exchange between blood and hepatic cells is inhibited by the interstitial ECM accumulated in the space of Disse, it is difficult for hepatic cells to supply nutrients and release toxic substances, and hepatic cells are also continuously damaged. While such a series of reactions are repeated, connective tissues are accumulated in liver tissue, causing liver fibrosis or liver cirrhosis.

These days, studies on liver fibrosis are being actively conducted in various ways, including studies on ECM, studies on the relationship between cells involved in ECM and various cells, the mechanism of cellular activation, various cytokines and antifibrogenic agents, and the like, and aim to develop a method for early diagnosis of liver fibrosis and an agent for treating liver fibrosis. Particularly, as it is known that the greatest cause of liver fibrosis and liver cirrhosis that involve the production of excessively accumulated connective tissues is the activation of hepatic stellate cells, studies on the development of drugs capable of inhibiting the activity and activation of stellate cells are being continuously conducted.

Drugs that are currently used are very diverse, but are mostly used for liver diseases such as hepatitis, and many of these drugs cause side effects in clinical applications or are costly.

Meanwhile, stem cells are cells which are capable of differentiating into a variety of cells constituting tissues of an organism, and generally refer to undifferentiated cells before differentiation, which can be obtained from respective tissues of an embryo, a fetus, and an adult body. The stem cells are characterized by differentiating into specific cells by a differentiation stimulus (environment); allowing proliferation (expansion) thereof by producing the same cells as themselves through cell division (self-renewal), unlike cells of which cell division has been ceased due to completion of differentiation; and having plasticity in differentiation since they can differentiate into other cells under different environments or by different differentiation stimuli.

The stem cells may be classified into pluripotent, multipotent, and unitent stem cells according to differentiation capability thereof. The pluripotent stem cells are pluripotent cells having totipotency to differentiate into all cells, and these include embryonic stem cells (ES cells), and induced pluripotent stem cells (iPS cells), etc. Adult stem cells may be examples of the multipotent and/or unipotent stem cells.

The embryonic stem cells are formed from the inner cell mass of blastocyte in early embryogenesis; have totipotency to differentiate into all cells so that they can differentiate into any kind of tissue cells; can be cultured in an immortal and undifferentiated state; and can be inherited to the next generation through preparation of germ cells, unlike the adult stem cells (Thomson et al., Science, 282: 1145-1147, 1998; Reubinoff et al., Nat. Biotechnol., 18: 399-404, 2000).

Human embryonic stem cells are prepared by isolating and culturing only the inner cell mass at the time of forming the a human embryo formation and, currently, the human embryonic stem cells prepared globally have been obtained from the frozen embryos remaining after sterilization operations. There have been various attempts to use pluripotent human embryonic stem cells that can differentiate into all cells as a cell therapy product; however, they have not yet completely overcome high barriers such as the risk of carcinogenesis and immunological rejection.

Tan, Z., Su, Z., Wu, R., Gu, B., Liu, Y., Zhao, X., & Zhang, M. (2011). Immunomodulative effects of mesenchymal stem cells derived from human embryonic stem cells in vivo and in vitro. Journal of Zhejiang University SCIENCE B, 12(1), 18-27*,* discloses an identical and clinically compliant MSC source derived from hESCs (hESC-MSCs) the immunomodulative effects of hESC-MSCs in vitro and in vivo for a carbon tetrachloride (CC14)-induced liver inflammation model. The hESC-MSCs therein is obtained via a process comprising: culturing undiferentialted hESCs with bFGF containing medium; pretreating cells with Rho-associated kinase inhibitor and inducing the cells to fibroblast-looking cells. These cells were tested for their surface markers and multilineage differentiation capability. hESC-MSCs were then administered to a murine carbon tetrachloride-induced liver inflammation model. A significant decrease in positive staining for CD3 was observed, indicating that the inflammatory infiltration into the injured area.

Recently, mesenchymal stem cells that have an immunoregulatory function and are free from the risk of tumorigenesis, have been presented as an alternative for solving such problems. The mesenchymal stem cells are multipotent cells which are capable of differentiating into adipocytes, osteocytes, chondrocytes, myocytes, neurocytes, cardiomyocytes, etc., and have been reported to have a function of regulating immune responses. The mesenchymal stem cells can be isolated and cultured from various tissues, but their capacity and cell surface markers are different from one another depending on the origins thereof. Therefore, it is not easy to clearly define the mesenchymal stem cells. However, the mesenchymal stem cells are generally defined by cells which can differentiate into osteocytes, chondrocytes and myocytes; have a spiral form; and express CD73(+), CD105(+), CD34(-), and CD45(-), which are basic cell surface markers.

Meanwhile, the minimal number (about 1×10⁹) of cells required in the fields of regenerative medicine and/or cell therapy needs to be satisfied, in order for the mesenchymal stem cells to be used as cell therapy products. However, the number of cells actually required is further increased, when considering experiments for setting proper conditions and standards.

The most ideal alternative to solve the above problems of the existing mesenchymal stem cell culturing system is to use human pluripotent stem cells to produce mesenchymal stem cells. However, so far, the induction of differentiation from human pluripotent stem cells into mesenchymal stem cells had required an induction procedure by a specific cytokine (e.g., BMP, bFGF), which costs much and needs control of concentration, or an induction procedure on xeno feeders (OP9 mouse cell lines) having the risk of xeno pathogen, and a sorting by a specific marker (e.g., CD73), thereafter. For these reasons, the mesenchymal stem cells have limitations in being used as ideal cell therapy products in the fields of regenerative medicine and cell therapy.

### DISCLOSURE OF INVENTION

### TECHNICAL PROBLEM

The present invention has been made in order to solve the above-described problems occurring in the prior art, and it is an object of the present invention to develop a pharmaceutical composition or functional health food for use in the prevention and/or treatment of liver fibrosis and liver cirrhosis, using mesenchymal stem cells derived from human embryonic stem cells. The present invention is defined in independent claim 1.

### TECHNICAL SOLUTION

To achieve the above object, the present invention provides a composition for use in preventing and/or treating liver fibrosis or liver cirrhosis, which contains mesenchymal stem cells derived from human embryonic stem cells as an active ingredient, wherein said mesenchymal stem cells are produced by a method comprising the following steps a) to c):
a) culturing human pluripotent stem cells in a basic fibroblast growth factor (bFGF)-free medium for 12-16 days to form embryoid bodies;
b) attaching the embryoid bodies of step a) to a culture dish and culturing the embryoid bodies in a medium comprising a basal DMEM (Dulbecco's Modified Eagle's Medium) supplemented with FBS (fetal bovine serum) to induce differentiation of embryoid bodies into mesenchymal stem cells; and
c) proliferatively culturing the mesenchymal stem cells of step b) using a culture medium containing human epidermal growth factor (hEGF), vascular endothelial growth factor (VEGF), human fibroblast growth factor-basic (hFGF-B), insulin-like growth factor (IGF-1), hydrocortisone and ascorbic acid, while maintaining the identity of the mesenchymal stem cells.

The present disclosure also provides a functional health food for alleviating and/or preventing liver fibrosis and liver cirrhosis, which contains mesenchymal stem cells derived from human embryonic stem cells as an active ingredient.

### ADVANTAGEOUS EFFECTS

The mesenchymal stem cells contained in the composition for use according to claim 1 are obtained by differentiation from all human pluripotent stem cells regardless of a difference in the genetic background thereof, and particularly, are induced to differentiate from human embryonic stem cells, and can be produced in large amounts by proliferative culture. In addition, the mesenchymal stem cells have an excellent effect on the prevention of liver fibrosis compared to conventional bone marrow-derived mesenchymal stem cells, and thus are effectively used as an active ingredient in herein disclosed, but not claimed, cell therapy products or functional health foods for preventing and treating liver fibrosis or liver cirrhosis.

### BRIEF DESCRIPTION OF THE DRAWINGS

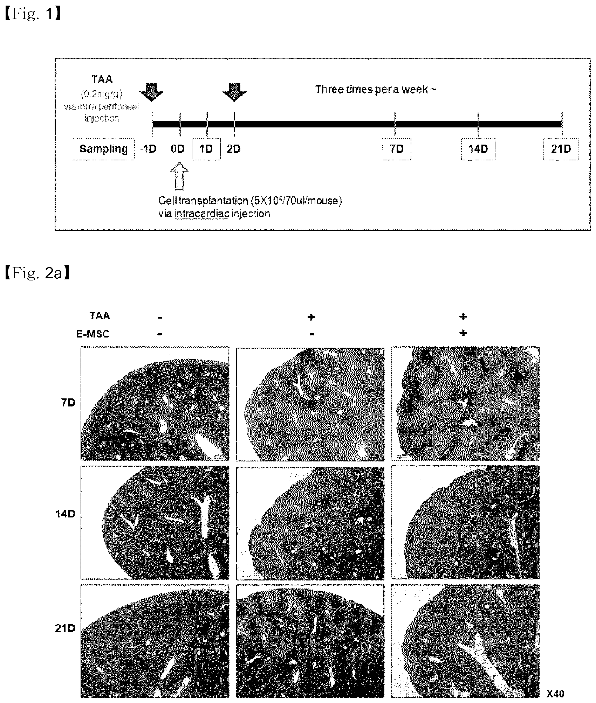

FIG. 1 is a schematic diagram that summarizes time intervals for the treatment of test animals with TAA and mesenchymal stem cells, and a sampling procedure.

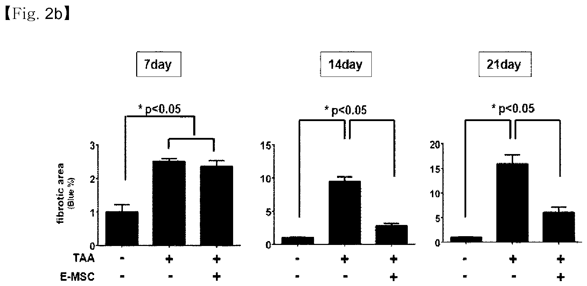

FIG. 2 shows the results of performing Masson's trichrome (MT) staining of the liver tissues of an untreated normal group (TAA - /E-MSC -), a test group (TAA + /E-MSC -) treated with TAA to induce liver fibrosis, and a test group (TAA + /E-MSC +) injected with mesenchymal stem cells after induction of liver fibrosis. Herein, FIG. 2a shows the results of microscopic observation of MT staining results, and FIG. 2b shows the results of numerically quantifying the degree of fibrosis.

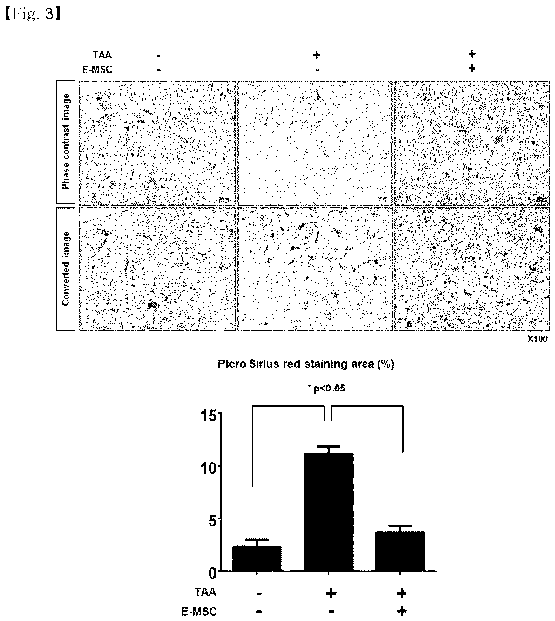

FIG. 3 shows the results of performing Pico-Sirius red staining of the liver tissues of an untreated normal group (TAA - /E-MSC -), a test group (TAA + /E-MSC -) treated with TAA to induce liver fibrosis, and a test group (TAA + /E-MSC +) injected with mesenchymal stem cells after induction of liver fibrosis.

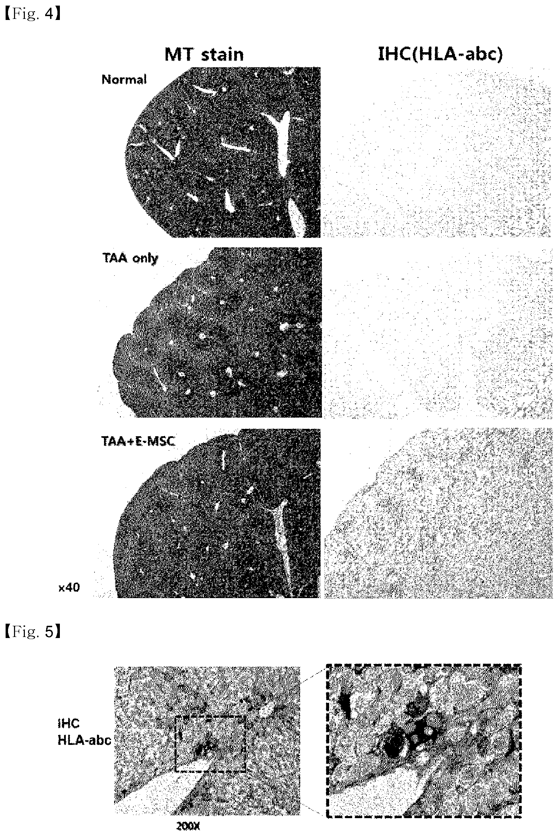

FIG. 4 shows the results of performing immunohistochemistry of an untreated normal group (Normal), a test group (TAA only) treated with TAA to induce liver fibrosis, and a test group (TAA + E-MSC) injected with mesenchymal stem cells after induction of liver fibrosis by TAA treatment.
FIG. 5 shows the results of magnifying and observing the portion that reacted with the human-specific antibody HLA-abc in the test group (TAA + E-MSC) injected with mesenchymal stem cells after induction of liver fibrosis by TAA treatment, among the results shown in FIG. 4.

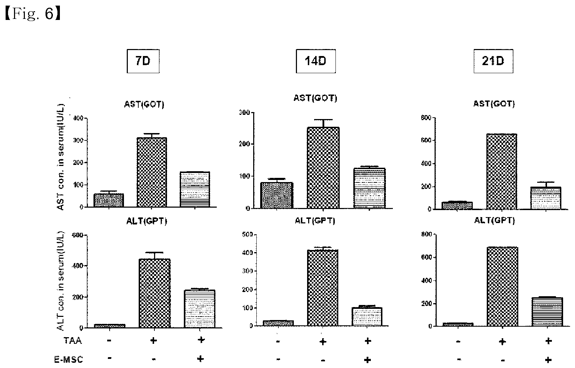

FIG. 6 shows the results of measuring hepatotoxicity parameters (AST and ALT) for the liver tissues of an untreated normal group (TAA - /E-MSC -), a test group (TAA + /E-MSC -) treated with TAA to induce liver fibrosis, and a test group (TAA + /E-MSC +) injected with mesenchymal stem cells after induction of liver fibrosis.

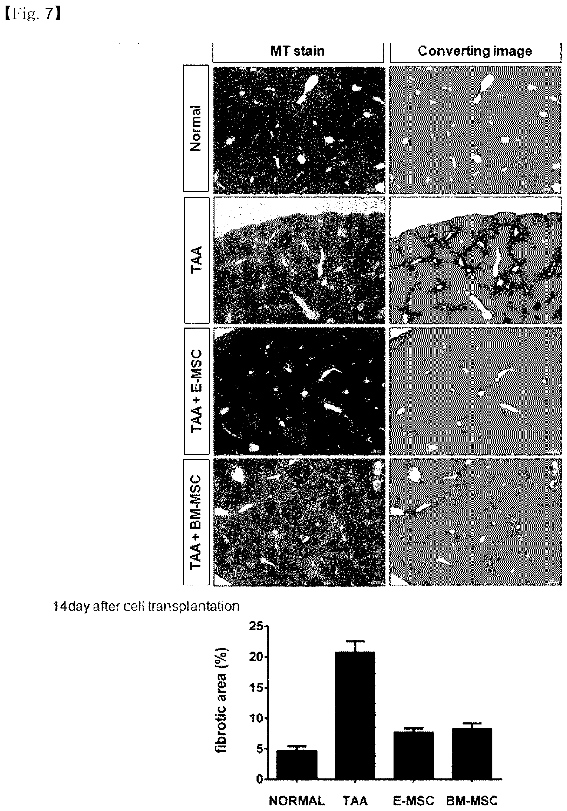

FIG. 7 shows the results of performing MT staining, observation and quantitative evaluation of the liver tissues of an untreated normal group (Normal), a test group (TAA) treated with TAA to inducer liver fibrosis, a test group (TAA + E-MSC) injected with human embryonic stem cell-derived mesenchymal stem cells after induction of liver fibrosis by TAA treatment, and a test group injected with bone marrow-derived mesenchymal stem cells after induction of liver fibrosis by TAA treatment.

### BEST MODE FOR CARRYING OUT THE INVENTION

The present inventors have produced a large amount of mesenchymal stem cells from human pluripotent stem cells, particularly human embryonic stem cells, and have found that the produced mesenchymal stem cells have a preventive effect against liver fibrosis, suggesting that mesenchymal stem cells derived from human embryonic stem cells can be effectively used as an active ingredient in a composition for protecting the liver and preventing and treating liver fibrosis or liver cirrhosis, thereby completing the present invention.

The present invention is defined in claim 1. Unclaimed aspects, such as the mesenchymal stem cells as such, or compositions as such, or alternative methods of obtaining the cells, are part of the disclosure and described hereinafter.

The present disclosure provides a composition for preventing and/or treating liver fibrosis or liver cirrhosis, which contains mesenchymal stem cells derived from human embryonic stem cells as an active ingredient.

The present disclosure also provides a method for treating liver fibrosis or liver cirrhosis, which comprises a step of administering mesenchymal stem cells derived from human embryonic stem cells to a subject.

The mesenchymal stem cells according to the present disclosure are preferably produced by a method comprising the following steps a) to c), but are not limited thereto: a) forming embryoid bodies from human pluripotent stem cells; b) attaching the embryoid bodies to a tissue culture dish, and inducing the attached embryoid bodies to spontaneously differentiate into mesenchymal stem cells; and c) maintaining and proliferatively culturing the mesenchymal stem cells resulting from induction of differentiation of the embryoid bodies.

In addition, the step of forming embryoid bodies from human pluripotent stem cells may be performed according to a general method known in the art. For example, this step can be performed by treating human pluripotent stem cells with protease and culturing the treated human pluripotent stem cells in a bFGF (basic Fibroblast Growth Factor)-free embryonic stem cell medium in a plate or suspension state.

Furthermore, the mesenchymal stem cells are preferably produced by a method comprising the following steps a) to c):
a) culturing human pluripotent stem cells for 12-16 days to form embryoid bodies;
b) culturing the embryoid bodies in a medium comprising a basal DMEM (Dulbecco's Modified Eagle's Medium) supplemented with FBS (fetal bovine serum) to induce differentiation of embryoid bodies into mesenchymal stem cells; and
c) proliferatively culturing the mesenchymal stem cells using a culture medium containing human epidermal growth factor (hEGF), vascular endothelial growth factor (VEGF), human fibroblast growth factor-basic (hFGF-B), insulin-like growth factor (IGF-1), hydrocortisone and ascorbic acid, while maintaining the identity of the mesenchymal stem cells.

As used herein, the term "stem cell" refers to a master cell that can reproduce indefinitely to form the specialized cells of tissues and organs. A stem cell is a developmentally pluripotent or multipotent cell. A stem cell can divide to produce two daughter stem cells, or one daughter stem cell and one progenitor ("transit") cell, which then proliferates into the tissue's mature, fully formed cells. Such stem cells can be classified in various ways. According to differentiation capability which is a method that is most frequently used for classification, stem cells can be divided into pluripotent stem cells capable of differentiating into three germ layers, multipotent stem cells capable of differentiating into one or more of the three germ layers, and unipotent stem cells capable of differentiating only into a certain germ layer.

As used herein, the term "pluripotent stem cells" refers to stem cells that have the potential to differentiate into any of the three germ layers of an organism, and generally includes embryonic stem cells and induced pluripotent stem cells (iPSs). Adult stem cells can be classified into multipotent stem cells and unipotent stem cells.

As used herein, the term "differentiation" refers to a phenomenon in which the structure or function of cells is specialized during the division, proliferation and growth thereof, that is, the feature or function of cell or tissue of an organism changes in order to perform work given to the cell or tissue. Generally, it refers to a phenomenon in which a relatively simple system is divided into two or more qualitatively different partial systems. For example, it means that a qualitative difference between the parts of any biological system, which have been identical to each other at the first, occurs, for example, a distinction, such as a head or a body, between egg parts, which have been qualitatively identical to each other at the first in ontogenic development, occurs, or a distinction, such as a muscle cell or a nerve cell, between cells, occurs, or the biological system is divided into qualitatively distinguishable parts or partial systems as a result thereof.

When pluripotent stem are suspension-cultured in a bFGF (basic Fibroblast Growth Factor)-free embryonic stem cell culture medium without a plate or support, differentiation derivatives can be produced. As reported in the art, differentiation derivatives that are produced by this method can differentiate into all types of cells required for formation of endoderm, mesoderm and ectoderm according to specific inducers, and this method is an *in vitro* method that is used to demonstrate the pluripotency of pluripotent stem cells.

As used herein, the term "cell therapeutic agent" refers to a drug used for the purpose of treatment, diagnosis and prevention, which contains a cell or tissue prepared through isolation from man, culture and specific operation (as provided by the US FDA). Specifically, it refers to a drug used for the purpose of treatment, diagnosis and prevention through a series of behaviors of in vitro multiplying and sorting living autologous, allogenic and xenogenic cells or changing the biological characteristics of cells by other means for the purpose of recovering the functions of cells and tissues.

The composition for preventing and/or treating liver fibrosis or liver cirrhosis according to the present disclosure which contains, as an active ingredient, mesenchymal stem cells derived from human embryonic stem cells, may be injected into the body of a patient as cells alone or after cell culture in an incubator. For example, a clinical method reported by Lindvall et al. (1989, Arch. Neurol. 46: 615-31) or Douglas Kondziolka (Douglas Kondziolka, Pittsburgh, 1998) may be used to administer the composition. The formulation of the composition may comprise, in addition to adipose, bone, muscle, neuron, cartilage and myocardial cells that initially differentiated from the mesenchymal stem cells derived from human embryonic stem cells, a pharmaceutically acceptable conventional carrier. For injection, the formulation may include a preservative, an analgesic, a solubilizer, a stabilizer or the like, and for topical administration, the formulation may include a base, an excipient, a lubricant, a preservative or the like.

A pharmaceutically acceptable carrier contained in the pharmaceutical composition of the present disclosure is typically used in the formulation. Examples thereof include lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrups, methyl cellulose, methylhydroxybenzoate, propylhydroxybenzoate, talc, magnesium stearate, and mineral oil, but are not limited thereto. The pharmaceutical composition of the present invention may further include lubricants, wetting agents, sweeteners, aromatics, emulsifiers, suspensions, and preservatives besides the above components.

The pharmaceutical composition according to the present disclosure may be prepared in single-dose forms or in multidose packages using a pharmaceutically acceptable carrier and/or excipient according to a method that may be easily carried out by those skilled in the art. Herein, the formulation of the pharmaceutical composition may be a solution, suspension or emulsion of the pharmaceutical composition in oil or aqueous medium, and may further comprise a dispersing agent or a stabilizer.

The pharmaceutical composition according to the present disclosure may be administered parenterally (e.g., intravenously, subcutaneously, intraperitoneally, or topically). Preferably, the pharmaceutical composition according to the present disclosure may be administered by intracardiac injection. The composition (e.g., injectable solution) for parenteral administration according to the present invention can be injected *in vivo* by dispersion and/or dissolution thereof in a pharmaceutically acceptable carrier, for example, sterile purified water, a buffer of about pH 7, or saline solution. The pharmaceutical composition of the present disclosure may include a conventional additive such as a preservative, a stabilizer or the like.

In addition, in the present disclosure the amount of mesenchymal stem cells injected is not specifically limited, but may be 10⁴ to 10¹⁰ cells/injection, preferably 10⁵ to 10⁹ cells/injection, more preferably 5×10⁷ cells/injection. The preferred dosage of the pharmaceutical composition of the present disclosure can be suitably selected depending on various factors, including formulation method, administration method, the patient's age, weight and gender, the severity of disease, diet, the route and period of administration, excretory speed, and response sensitivity.

As used herein, the term "preventing" refers to all actions that inhibit liver fibrosis or liver cirrhosis or delay the progression of liver fibrosis or liver cirrhosis by administering the composition of the present disclosure As used herein, the term "preventing" or "prevention" refers to all kinds of activities associated with the inhibition or delay of liver fibrosis or liver cirrhosis by administering the pharmaceutical composition of the present disclosure As used herein, the term "treating" or "alleviating" refers to all kinds of activities that alleviate or beneficially change liver fibrosis or liver cirrhosis by administering the composition of the present disclosure.

Unless defined otherwise, the terms used herein have the same meanings as commonly understood by one of ordinary skill in the art to which the invention pertains.

The pharmaceutical composition of the present disclosure may be administered in combination with a therapeutic method which has been typically used to treat or prevent liver fibrosis and liver cirrhosis.

The present disclosure also provides a functional health food for alleviating and/or preventing liver fibrosis and liver cirrhosis, which contains, as an active ingredient, mesenchymal stem cells derived from human embryonic stem cells.

If the human embryonic stem cell-derived mesenchymal stem cells according to the present disclosure are used as a food additive, these cells may be added alone or may be suitably used together with other foods or food ingredients according to a conventional method. The content of the active ingredient in the composition can be suitably determined depending on the purpose of use (prophylactic, health or therapeutic treatment). Generally, the human embryonic stem cell-derived mesenchymal stem cells may be added in an amount of 0.0001-30 wt%, preferably 0.1-10 wt%, based on the total weight of raw materials, during the production of a food or a beverage, but the amount of human embryonic stem cell-derived mesenchymal stem cells added can be suitably controlled depending on the intended use thereof. There is no particular limit to the kind of food. Examples of foods to which the human embryonic stem cell-derived mesenchymal stem cells may be added include meats, sausages, bread, chocolate, candies, snack, confectionery, pizza, noodles, gum, dairy products including ice cream, various soups, beverages, teas, drinks, alcoholic beverages, and multi-vitamin preparations.

### EXAMPLES

Hereinafter, the present invention will be described in further detail with reference to examples. It will be obvious to a person having ordinary skill in the art that these examples are illustrative purposes only and are not to be construed to limit the scope of the present invention. Thus, the substantial scope of the present invention will be defined by the appended claim.

### Example 1: Production of Mesenchymal Stem Cells Derived from Human Embryonic Stem Cells

Mesenchymal stem cells derived from human embryonic stem cells according to the present disclosure were constructed and cultured according to the following method.

### (1) Formation of Embryoid Bodies

Human embryonic stem cells (Oriental #1, male, STO feeder) maintained in an undifferentiated state in the Seoul National University Hospital were treated with dispase (2 mg/ml) and isolated by micro-operations, followed by suspension culture in a bFGF-free embryonic stem cell medium for 14 days.

### (2) Induction of Differentiation into Mesenchymal Stem Cells

Embryoid bodies made by 14 days of suspension culture were attached to a tissue culture dish, and then spontaneous differentiation of the attached embryoid bodies into mesenchymal stem cells was induced. While the embryoid bodies were cultured in a DMEM (Dulbecco's Modified Eagle's Medium) containing 10% (v/v) of FBS (fetal bovine serum) for 16 days, induction of differentiation of the embryoid bodies into mesenchymal stem cells was observed.

### (3) Maintenance and Proliferative Culture of Mesenchymal Stem Cells Resulting from Induction of Differentiation

Mesenchymal stem cells that differentiated from the embryoid bodies by 16-day culture after attachment of the embryoid bodies in Example 1-(2) were dissociated into single cells by treatment with Trypsin-EDTA (0.25% Trypsin with EDTA 4Na), and then attached again to a tissue culture dish. Next, the cells were maintained and proliferatively cultured in 500 mℓ of a medium (EGM-2MV, CC4147, Lonza) comprising 470mℓ of a basal medium, 0.5mℓ of hEGF (human Epidermal Growth Factor), 0.5 mℓ of VEGF (Vascular Endothelial Growth Factor), 2mℓ of hFGF-B (human Fibroblast Growth Factor-basic), 0.5 mℓ of IGF-1 (Insulin-like Growth Factor), 0.2 mℓ of hydrocortisone and 0.5 mℓ of ascorbic acid at 37°C.

### Example 2: Effect of Human Embryonic Stem Cell-Derived Mesenchymal Stem Cells on Prevention of Liver Fibrosis

In order to examine whether the mesenchymal stem cells produced in Example 1 have the effect of preventing liver fibrosis, test animals were treated with thioacetamide (TAA; C₂H₅NS) known as a liver fibrosis inducer to induce liver cirrhosis. In addition, the mesenchymal stem cells were injected intracardiacally into the test animals, and whether the liver cirrhosis in the animals was alleviated was examined.

Thioacetamide (TAA; C₂H₅NS), a kind of liver toxin similar to CCl₄ or D-galactosamine that induces cell necrosis or death, is known to interfere with RNA migration from the nucleus into the cytoplasm to thereby induce damage to the cell membrane and the structural and functional destruction of cells.

Specifically, immunodeficient mice (nude mice, male, 8-12 weeks old, Orient Bio) were injected intraperitoneally with TAA (in 0.9% normal saline solution) at a dose of 200 mg/kg body weight, three times a week for 3 weeks, to induce fibrosis. At 24 hours after the first treatment with TAA, 5 x 10⁴ cells of the mesenchymal stem cells produced in Example 1 were administered to the mice once by intracardiac injection, and at 24 hours after the final treatment with TAA, tissue was sampled from the mice and analyzed (FIG. 1). The sampled tissue was ① observed visually to determine the degree of aggregation of the tissue, ② analyzed by fibrosis (Masson's trichrome) staining using a tissue slide in order to compare the degree of alleviation of fibrosis, ③ analyzed by immunohistochemistry using the human cell-specific expressing factor HLA-abc in order to determine whether the injected mesenchymal stem cells remained, and ④ measured for hepatotoxicity markers (AST and ALT).

### 2-1: Fibrosis (Masson's Trichrome (MT)) Staining

When fibrosis of liver cells progresses, collagen is synthesized to fill a damaged site, and MT stain reacts with collagen to develop a blue color that labels a fibrotic portion. The obtained tissue sample was analyzed by MT staining to examine the degree of fibrosis.

Specifically, MT staining was performed in the following manner.

The tissue slide fixed with paraffin was deparaffinized, and then fixed with Bouin's solution and stained with Weigert's iron hematoxylin/Biebrich scarlet-acid fuchsin-aniline blue solution or 2% light green, followed by observation. Fibrotic tissue appears as a blue color, and cytoplasm, muscle and keratin appear as a red color, and the cell nucleus appears as a dark brown color.

As a result, as shown in FIGS. 2a and 2b, the group (TAA + /E-MSC -) treated with TAA to induce liver fibrosis showed an increase in the blue area compared to the untreated normal group (TAA - /E-MSC -), and the group (TAA + /E-MSC +) treated with the mesenchymal stem cells showed a significant decrease in the blue area compared to the TAA + /E-MSC - group.

In addition, as can be seen in FIG. 2a, it was observed that, in the tissue treated with TAA to induce liver fibrosis, a lump and surface irregularities were formed due to liver fibrosis, and this phenomenon was alleviated by injection of the mesenchymal stem cells.

### 2-2. Picro-Sirius Red Staining

When fibrosis of liver cells progresses, collagen is synthesized to fill a damaged site, and MT stain reacts with collagen to develop a blue color that labels a fibrotic portion. The obtained tissue sample was analyzed by Picro-Sirius red staining to examine the degree of fibrosis.

Picro-Sirius red staining is a method enabling fibrosis to be examined, like MT staining. These two staining methods were used to examine the degree of inhibition of fibrosis in duplicate.

As a result, Picro-sirius red staining was performed in the following manner.

Paraffin tissue slides were deparaffinized, and then stained with Picro-sirius red for 1 hour and washed with water, followed by drying and fixing.

As a result, as can be seen in FIG. 3, the group (TAA + /E-MSC -) treated with TAA to induce liver fibrosis showed an increase in the red area compared to the untreated normal group (TAA - /E-MSC -), and the group (TAA + /E-MSC +) treated with the mesenchymal stem cells showed a significant decrease in the red area compared to the TAA + /E-MSC - group.

### 2-3: HLA-abc IHC Staining

In order to examine whether the liver fibrosis alleviation effect confirmed in Examples 2-1 and 2-2 was attributable to the human mesenchymal stem cells injected into the animals, immunohistochemistry was performed using the human cell-specific expressing factor HLA-abc, thereby determining whether the mesenchymal stem cells remained in the tissue sample showing the liver fibrosis alleviation effect.

Specifically, IHC staining using the human-specific expressing antigen HLA-abc was performed in the following manner.

After an antigen-antibody reaction, the tissue sample was treated with biotin secondary antibody. After treatment with streptavidin-HRP, the tissue sample was stained with substrate-charmogen solution to develop a brown color. Specifically, IHC staining was performed using an IHC kit of a Vectorlab R.T.U kit (Vector, cat# PK-7800), and HLA-abc purchased from Abcam was used. In addition, pan-specific universal secondary antibody included in the kit was used as secondary antibody, and DAB (Vectorlab NOVA red DAB substrate kit cat# SK-4800) was used to develop color.

As a result, as can be seen in FIG. 4, in the liver tissues of the normal group and the group (TAA only) treated with TAA to induce liver fibrosis, a brown color reaction was not observed, but in the tissue of the group (TAA + E-MSC) injected with the mesenchymal stem cells after TAA treatment to induce liver fibrosis, a brown color reaction was observed, indicating that an antigen-antibody reaction occurred. Magnified observation of the tissue of the group (TAA + E-MSC) showed an engraft of the treated mesenchymal stem cells (FIG. 5) .

### 2-4: Measurement of Hepatotoxicity parameters (AST and ALT)

In order to further confirm the effect of alleviating hepatotoxicity caused by the progression of liver fibrosis as confirmed in Examples 2-1 to 2-3, AST (aspartate aminotransferase) and ALT (alanine aminotransferase), which are hepatotoxicity parameters, were measured in the untreated normal group (TAA - /E-MSC -), the test group (TAA + /E-MSC -) treated with TAA to induce liver fibrosis, and the test group (TAA + /E-MSC +) injected with the mesenchymal stem cells after induction of liver fibrosis by TAA treatment. Aminotransferase is an enzyme present in any tissue, and intracellular aminotransferase activity is higher than serum aminotransferase activity. Thus, if tissue is damaged, the enzyme aminotransferase is released into blood so that blood aminotransferase activity increases. However, because the enzyme has a high molecular weight (about 100,000 Da), the migration of the enzyme from damaged cells into blood is hindered. Thus, if livers, myocardia, muscles and blood cells, from which the enzyme is easily released into blood, are damaged, serum aminotransferase activity increases, but if other organs are damaged, serum aminotransferase activity does not substantially increase.

AST and ALT were measured using a known method. Specifically, a method was used in which enzymatic activities are numerically quantified based on changes in color development reagents added to reactions that occur during degradation of AST and ALT substrates in serum separated from collected blood.

As a result, as can be seen in FIG. 6, on day 21, AST and ALT in the group treated with TAA to induce liver fibrosis significantly increased, whereas AST and ALT in the group injected with the mesenchymal stem cells after induction of liver fibrosis significantly decreased.

This suggests that the mesenchymal stem cells of the present disclosure exhibit excellent effects on the prevention and treatment of liver fibrosis.

### Example 3: Comparison of Effects between Human Embryonic Stem Cell-Derived Mesenchymal Stem Cells and Bone Marrow-Derived Mesenchymal Stem Cells

The effect on alleviation of liver fibrosis was evaluated comparatively between the human embryonic stem cell-derived mesenchymal stem cells produced in Example 1 and bone marrow-derived mesenchymal stem cells.

MT staining was performed in the same manner as described in Example 2-1, except that bone marrow-derived mesenchymal stem cells were used instead of the human embryonic stem cell-derived mesenchymal stem cells.

As a result, as can be seen in FIG. 7, the test group (TAA + E-MSC) injected with the human embryonic stem cell-derived mesenchymal stem cells after induction of liver fibrosis by TAA treatment showed a decrease in the blue-stained area compared to the test group (TAA + BM-MSC) injected with bone marrow-derived mesenchymal stem cells. This suggests that the human embryonic stem cell-derived mesenchymal stem cells of the present invention have a better effect on the alleviation of liver fibrosis.

### INDUSTRIAL APPLICABILITY

According to the present invention, the mesenchymal stem cells of the present disclosure have an excellent effect on the prevention of liver fibrosis compared to conventional bone marrow-derived mesenchymal stem cells, and thus it is possible to provide in cell therapy products or functional health foods for preventing and treating liver fibrosis or liver cirrhosis.

## Claims

1. A composition for use in preventing and/or treating liver fibrosis or liver cirrhosis, which contains, as an active ingredient, mesenchymal stem cells derived from human embryonic stem cells, wherein said mesenchymal stem cells are produced by a method comprising the following steps a) to c):
a) culturing human pluripotent stem cells in a basic fibroblast growth factor (bFGF)-free medium for 12-16 days to form embryoid bodies;
b) attaching the embryoid bodies of step a) to a culture dish and culturing the embryoid bodies in a medium comprising a basal DMEM (Dulbecco's Modified Eagle's Medium) supplemented with FBS (fetal bovine serum) to induce differentiation of embryoid bodies into mesenchymal stem cells; and
c) proliferatively culturing the mesenchymal stem cells of step b) using a culture medium containing human epidermal growth factor (hEGF), vascular endothelial growth factor (VEGF), human fibroblast growth factor-basic (hFGF-B), insulin-like growth factor (IGF-1), hydrocortisone and ascorbic acid, while maintaining the identity of the mesenchymal stem cells.

## Patentansprüche

1. Zusammensetzung zur Verwendung bei der Prävention und/oder Behandlung von Leberfibrose oder Leberzirrhose, die als Wirkstoff mesenchymale Stammzellen enthält, die von humanen embryonalen Stammzellen abstammen, wobei die mesenchymalen Stammzellen nach einem Verfahren hergestellt sind, das die folgenden Schritte a) bis c) umfasst:
a) Kultivieren von humanen pluripotenten Stammzellen in einem Medium, das frei von basischem Fibroblasten-Wachstumsfaktor (bFGF) ist, während 12-16 Tagen unter Bildung von Embryoid Bodies;
b) Anheften der Embryoid Bodies von Schritt a) an eine Kulturschale und Kultivieren der Embryoid Bodies in einem Medium, das DMEM-Grundmedium (Dulbeccos modifiziertes Eagle-Medium), welches mit FBS (fetalem Kälberserum) ergänzt ist, umfasst, zur Induktion der Differenzierung der Embryoid Bodies zu mesenchymalen Stammzellen; und
c) proliferative Kultur der mesenchymalen Stammzellen von Schritt b) unter Verwendung eines Kulturmediums, das humanen Epidermis-Wachstumsfaktor (hEGF), Gefäßendothel-Wachstumsfaktor (VEGF), humanen Fibroblasten-Wachstumsfaktor-Basisch (hFGF-B), insulinartigen Wachstumsfaktor (IGF-1), Hydrocortison und Ascorbinsäure enthält, während die Identität der mesenchymalen Stammzellen aufrechterhalten wird.

## Revendications

1. Composition à utiliser dans la prévention et/ou le traitement de la fibrose hépatique ou de la cirrhose du foie, contenant comme principe actif des cellules souches mésenchymateuses dérivées de cellules souches embryonnaires humaines, dans laquelle lesdites cellules souches mésenchymateuses sont produites par un procédé comprenant les étapes suivantes a) à c) consistant à :
a) cultiver des cellules souches pluripotentes humaines dans un milieu exempt de facteur de croissance des fibroblastes basique (bFGF) pendant 12-16 jours pour former des corps embryoïdes,
b) attacher lesdits corps embryoïdes de l'étape a) à une boîte de culture, et cultiver les corps embryoïdes dans un milieu comprenant un milieu de base DMEM (milieu Eagle modifié de Dulbecco) enrichi de SVF (sérum de veau fœtal) pour induire la différentiation de corps embryoïdes en cellules souches mésenchymateuses, et
c) cultiver de manière proliférative les cellules souches mésenchymateuses de l'étape b) en utilisant un milieu de culture contenant du facteur de croissance épidermique humain (hEGF), du facteur de croissance endothélial vasculaire (VEGF), du facteur de croissance des fibroblastes humain-basique (hFGF-B), du facteur de croissance analogue à l'insuline (IGF-1), de l'hydrocortisone, et de l'acide ascorbique, tout en maintenant l'identité des cellules souches mésenchymateuses.
